# EUROPEAN PATENT SPECIFICATION

(11) **EP 3 068 746 B1**
(45) Date of publication and mention of the grant of the patent: **04.12.2019**
(21) Application number: 14747585.9
(22) Date of filing: 29.07.2014
(51) Int. Cl.: C07B 57/00, C07C 209/88, C07C 211/42, C07C 233/05, C07C 233/47

(54) **PROCESS FOR THE PREPARATION OF ENANTIOMERICALLY PURE 1-AMINOINDAN**
VERFAHREN ZUR HERSTELLUNG VON ENANTIOMERENREINEM 1-AMINOINDAN
PROCÉDÉ DE PRÉPARATION DE 1-AMINOINDANE ÉNANTIOMÉRIQUEMENT PUR

(30) Priority: 15.11.2013 SI 201300386
(43) Date of publication of application: 21.09.2016
(73) Proprietor: Farma GRS, d.o.o., 8000 Novo mesto (SI)
(72) Inventor: PRUDIC, Darja, 8000 Novo mesto (SI); RUZIC, Milos, 3000 Celje (SI); KLJAJIC, Alen, 3000 Celje (SI); PECAVAR, Anica, 8000 Novo mesto (SI); PLEVNIK, Miha, 1295 Ivancna Gorica (SI); KUFNER, Monika, 8000 Novo mesto (SI)
(74) Representative: Uexküll & Stolberg
(86) International application number: PCT/EP2014/066308
(87) International publication number: WO 2015/070995

(56) References cited:
- WO-A1-2012/116752

## Description

### Field of the invention

The present invention relates to an improved process for the preparation of enatiomerically pure (R)-1-aminoindan which is a valuable intermediate in the process for preparation of rasagiline. Moreover, the invention relates to diastereomeric salts of 1-aminoindan which are useful in the synthesis of (R)-1-aminoindan.

### Background of the invention

Rasagiline is a common name for (*R*)-*N*-(prop-2-ynyl)-2,3-dihydro-1*H-*inden-1-amine, a compound of formula (I):

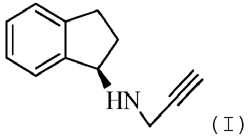

Rasagiline functions as a selective, irreversible MAO-B inhibitor indicated for the treatment of signs and symptoms of idiopathic Parkinson's disease as initial monotherapy and as adjunct therapy to levodopa. The selectivity for inhibiting MAO-B diminishes in a dose-related manner. MAO, a flavin-containing enzyme, is classified into two major molecular species, A and B, and is localized in mitochondrial membranes throughout the body in nerve terminals, brain, liver and intestinal mucosa. MAO regulates the metabolic degradation of catecholamines and serotonin in the CNS and peripheral tissues. MAO-B is the major form in the human brain. In *ex vivo* animal studies in brain, liver and intestinal tissues, rasagiline was shown to be a potent, irreversible monoamine oxidase type B (MAO-B) selective inhibitor. Rasagiline at the recommended therapeutic dose was also shown to be a potent and irreversible inhibitor of MAO-B in platelets. The precise mechanisms of action of rasagiline are unknown. One mechanism is believed to be related to its MAO-B inhibitory activity, which causes an increase in extracellular levels of dopamine in the striatum. The elevated dopamine level and subsequent increased dopaminergic activity are likely to mediate rasagiline's beneficial effects seen in models of dopaminergic motor dysfunction.

Several routes of synthesis have been reported for the preparation of rasagiline. The processes can generally be divided into two groups, namely (i) processes where racemic rasagiline is resolved in the last step of the synthesis and (ii) processes where the chiral center is introduced in an earlier step of synthesis. Since half of the product is discarded when using the enantiomeric resolution processes in the last step of synthesis, it is desirable to include chiral centers at an earlier stage of the synthesis thus resulting in more economical synthesis and better yields.

Rasagiline was first disclosed in EP 436492 which describes a method reacting (R)-1-aminoindan and propargyl chloride. The document teaches that (R)-1-aminoindan could be prepared by resolution of a racemic mixture of R- and S-enatiomers e.g. by formation of diastereomeric salts with chiral acids.

Reaction of a racemic base with an optically active acid gives a pair of diastereomeric salts. The members of this pair exhibit different physicochemical properties (e.g. solubility, melting point, boiling point, adsorption, phase distribution) and can be separated due to these differences. The most important method for the separation of enantiomers is crystallization.

In the prior art documents there are reported several processes for preparation of (R)-1-aminoindan using fractional crystallization of its diastereomeric salts.

In Boll. Chim. Farm., 115, p. 489-500, (1976) a method using N-acetyl-L-leucine as a resolving agent in an aqueous solution is disclosed. However, the R-isomer with the desired enantiomeric purity was recovered from mother liquors after 100 consecutive crystallizations. Consequently, also the yields are minimal.

In Biochemistry, 19, p. 2133-2139, (1980) a process using L-malic acid in absolute ethanol is disclosed. Four crystallizations from 96% ethanol have been required. Therefore, the process suffers from a very low yield and from being time consuming.

US 4833273 discloses a process for resolution of 1-aminoindan using (R)-N-acetyl-3,4-dimethoxyphenylalanine as resolving agent. The drawback of the process is that the unwanted diastereomeric salt (R,S) is precipitated first from the reaction mixture and that the desired salt (R,R) is recovered by concentrating and cooling mother liquors whereby more impure product was produced. This is also evident from the physical data, such as the melting point and the specific rotation, reported for the product of the first crop and the second crop which substantially differ from each other. In addition to that, the resolving agent used is very expensive and, therefore, not desirable for use in a large scale processes.

In WO 2009/147430 a process which uses 2,3,4,6-di-isopopylidene-2-keto-L-gulonic acid as a resolving agent is disclosed. The diasteromeric salt formation is performed in methanol. Recrystallization takes place in a solvent mixture of methanol and water. Enantiomeric purities for resolving processes exemplified in the document are about 96%. However, in processes for the preparation pharmaceutical active ingredients an enantiomeric purity of more than 98% is usually required. Another drawback of this process is the relatively high price of the reagent.

In WO 2012/116752 resolution of 1-aminoindan using L(+)-aspartic acid, L(-)-malic acid and (2R, 3R)-tartaric acid in methanol is disclosed. In case of resolution with L(+)-aspartic acid and (2R,3R)-tartaric acid, enantiomeric purities of less than 94% are reported. L(-)-malic acid is selected as preferred resolution agent providing product with an optical purity of 99.75% (HPLC), but the yield was lower than in case of the other two acids.

Other known methods include derivatization of 1-aminoindan prior to resolution. Such processes are however disadvantageous as several protection and deprotection steps are required during the process, which results in lower yield and more possibilities for formation of process impurities.

Preparation of optically pure compounds is one of the most important aims both for pharmaceutical practice and research. Actually, the resolution of racemic compounds (1:1 mixture of molecules having mirror-image relationship) still remains the most common method for producing pure enantiomers on a large scale. In these cases the enantiomeric mixtures or a sort of their derivatives are separated directly. This separation is based on the fact that the enantiomeric ratio in the crystallized phase differs from the initial composition. In this way, obtaining pure enantiomers requires one or more re-crystallizations.

Thus, there is still a need for an improved, practical, economical synthesis method for preparing (R)-1-aminoindan in high yields and at the same time high optical purity. Besides, this method should be applicable to scale up for industrial production.

### Figures

Figure 1 shows an X-ray diffraction pattern (XRDP) of racemic 1-aminoindan N-acetyl-L-glutaminate (1:1), i.e. (R,S)-1-aminoindan N-acetyl-L-glutaminate (1:1).
Figure 2 shows a FT-IR spectrum of racemic 1-aminoindan N-acetyl-L-glutaminate (1:1), i.e. (R,S)-1-aminoindan N-acetyl-L-glutaminate (1:1).
Figure 3 shows an X-ray diffraction pattern (XRDP) of optically pure (R)-1-aminoindan N-acetyl-L-glutaminate (2:1).
Figure 4 shows a FT-IR spectrum of optically pure (R)-1-aminoindan N-acetyl-L-glutaminate (2:1).

### Summary of the invention

The present invention relates to a process for preparation of optically pure (R)-1-aminoindan using N-acetyl-L-glutamic acid, and in particular to a process for preparing optically pure (R)-1-aminoindan by a diastereomeric resolution of 1-aminoindan using N-acetyl-L-glutamic acid as a resolving agent.

Another object of the invention relates to the diastereomeric salt of R-1-aminoindan with N-acetyl-L-glutamic acid, its use in the process for the preparation of rasagiline.

Yet another object of the invention is a process for racemization of (S)-1-aminoindan which is recovered from the mother liquor after diastereomeric salts separation.

A preferred embodiment of the process of the present invention is outlined in the following scheme:

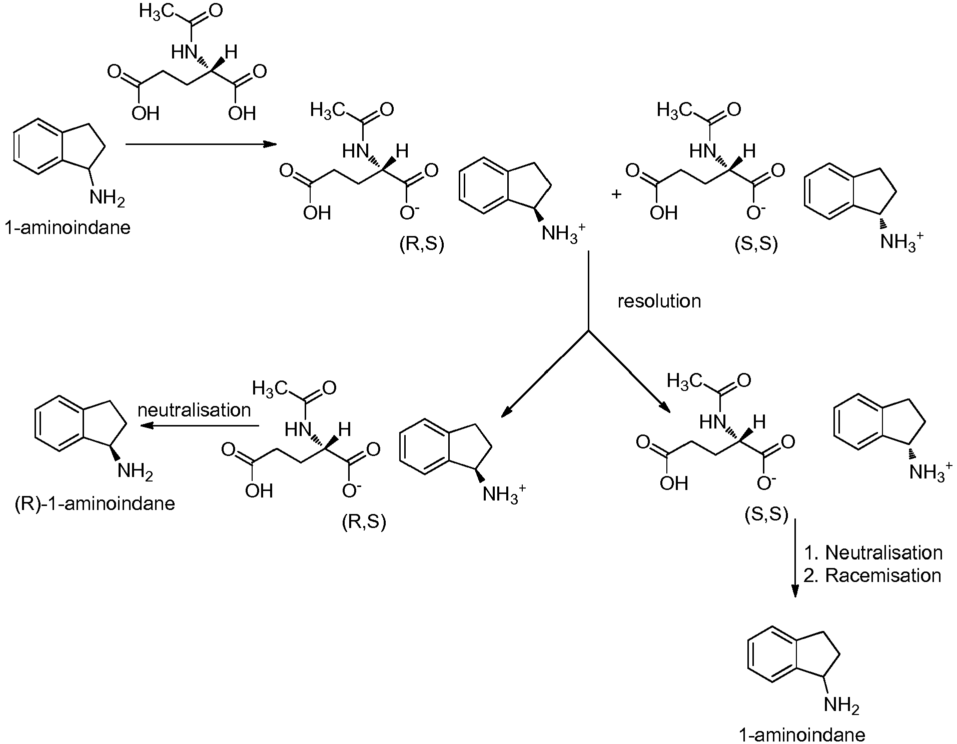

### Detailed description of the invention

In a first aspect, the present invention relates to a new process for the manufacture of optically pure (R)-1-aminoindan, which comprises a resolution of racemic 1-aminoindan using N-acetyl-L-glutamic acid as a resolving agent. According to a preferred embodiment, the process of the present invention comprises the following steps:
(i) formation of a diastereomeric salt of 1-aminoindan with N-acetyl-L-glutamic acid,
(ii) separating the diastereomeric salt in a first solvent system at a specified temperature range by means of fractional crystallization,
(iii) optionally, the diastereomeric salt thus obtained is crystallized in a second solvent system to get desired optical purity, and
(iv) neutralization of diastereomeric salt with desired optical purity to obtain optically pure (R)-1-aminoindan.

The term "optically pure" means that the desired enantiomeric form comprises less than 10 area % of unwanted enantiomeric form, preferably less than 5 area %, more preferably less than 2 area %, most preferably less than 1 area %; even most preferably less than 0.5 area %. Enantiomeric purity has been determined by means of HPLC analysis.

The term "desired optical purity" is equivalent to the term "optically pure".

As used herein, the term "racemic 1-aminoindan" refers to a mixture of (R)-1-aminoindan and (S)-1-aminoindan, having an (S):(R) ratio ranging from about 65:35 to about 35:65. Preferably, the racemic 1-aminoindan has an (S):(R) ratio ranging from about 55:45 to about 45:55. More preferably, the racemic 1-aminoindan has an S:R ratio ranging from about 52:48 to about 48/52. Even more preferably, the racemic 1-aminoindan has an S:R ratio ranging from about 51:49 to about 49:51. Most preferably, the racemic 1-aminoindan has an S:R ratio of about 1:1. Preferably, racemic 1-aminoindan is used as starting material in step (i) of the process of the present invention.

N-acetyl-L-glutamic acid is a diacid comprising two carboxylic acid groups. Therefore, according to the process of the present invention two types of diastereomeric salts could be formed, namely an acidic salt, wherein the molar ratio of 1-aminoindan to N-acetyl-L-glutamic acid is 1:1, or a neutral salt, wherein the molar ratio of 1-aminoindan to N-acetyl-L-glutamic acid is 2:1.

The formation of diastereomeric salts of 1-aminoindan with N-acetyl-L-glutamic acid according to step (i) may be carried out by combining racemic 1-aminoindan with N-acetyl-L-glutamic acid in a solvent and subsequent recovery of the salt formed.

The molar ratio of racemic 1-aminoindan to the N-acetyl-L-glutamic acid may be from 1:0.5 to 1:1.3, preferably from 1:0.6 to 1:1.1, most preferably from 1:0.7 to 1:1.

In a preferred embodiment, the salt formation in step (i) is carried in the presence of a solvent. The solvent used in step (i) may be selected from the group comprising alcohols, such as methanol, ethanol, 1-propanol and 2-propanol; ethers, such as diethyl ether, methyl ethyl ether, methyl tert-butyl ether (MTBE), isobutyl methyl ether (IBME), isopropyl ether, cyclopentyl methyl ether; water; hydrocarbons such as hexane, cyclohexane, heptane, octane, or a mixture thereof. Preferably, the solvent mixture of an alcohol and an antisolvent such as ether or hydrocarbon is used, more preferably mixture of ethanol with a solvent selected from diethyl ether, methyl ethyl ether, methyl tert-butyl ether (MTBE), isobutyl methyl ether (IBME), isopropyl ether, cyclopentyl methyl ether, hexane, cyclohexane or heptane, is used. Most preferably, a mixture of ethanol with methyl tert-butyl ether is used.

The diastereomeric salt formation (step (i)) as well as the resolution (steps (ii) and (iii)) may be carried out in the same solvent system or in a different solvent system. The first solvent system used in step (ii) and the second solvent system used in step (iii) may be identical or different.

In step (ii), a resolution of the diastereomeric salts obtained in step (i) takes place, i.e. the diastereomeric salts obtained in step (i) are separated by crystallization. In particular, the unwanted diastereomeric salts, i.e. salts comprising (S)-1-aminoindan or a mixture of salts enriched with (S)-1-aminoindan, remain in the mother liquor after crystallization and the desired diastereomeric salts, i.e. those comprising (R)-1-aminoindan or enriched with (R)-1-aminoindan, precipitate during crystallization. Likewise, in optional step (iii), a resolution of the precipitated diastereomeric salts obtained in step (ii) takes place.

The solvent system preferably used in the resolution steps (ii) and (iii) is a combination of alcoholic solvents such as methanol, ethanol, 1-propanol and 2-propanol with 0-10% (v/v) of dipolar solvents such as N,N-dimethylformamide, N,N-dimethylacetamide, N-methyl-2-pyrrolidone; dimethylsulfoxide or water. Preferably, the solvent system used in steps (ii) and (iii) comprises ethanol with 0-10% (v/v) of water. More preferably, the first solvent system comprises ethanol. It is also preferred that the first solvent system comprises ethanol and the second solvent system comprises a mixture of ethanol with 0.1-3% (v/v) of water. Even more preferably, the second solvent system comprises a mixture of ethanol with 0.5-2% (v/v) of water, and most preferably with 1% (v/v) of water.

Optimal solute to solvent ratios used in step (ii) can include 1g of the solute to 10 to 50 ml, preferably 1g of the solute to 20 to 40 ml of the first solvent system, whereas in step (iii) the preferred ratio includes 1g of the solute to 50 to 100 ml of the second solvent mixture, preferably 1g of the solute to 60 to 80 ml of the second solvent mixture.

The term "solute" relates to a diastereomeric salt of 1-aminoindan with N-acetyl-L-glutamic acid, which needs to be further optically purified.

The term "diastereomeric salt of 1-aminoindan with N-acetyl-L-glutamic acid, which needs to be further optically purified" means that the desired enantiomeric form comprises more than 0.5 area % of the unwanted enantiomeric form, preferably more than 1 area %, more preferably more than 2 area %, most preferably more than 5 area %, even most preferably more than 10 area % of the unwanted enantiomeric form. Enantiomeric purity has been determined by means of HPLC analysis.

The temperature can also play a key role in the resolution process for obtaining the optically pure (R)-1-aminoindan. The inventors have found out that if the resolution is carried out at a temperature of 5°C-80°C, the desired product is obtained in a higher optical purity. The best results can be achieved in cases where crystallization is initiated at a temperature between 20-65°C, preferably at 30° to 50°C, most preferably at 35°C to 45°C.

In order to carefully control the temperature of the onset of crystallization in steps (ii) or (iii), the crystallization is preferably initiated by addition of seeding crystals of a diastereomeric salt of (R)-1-aminoindan and N-acetyl-L-glutamic acid. The seeding crystals may be added at a temperature of 20 to 65°C, preferably at 30° to 50°C, most preferably at 35°C to 45°C. If seeding crystals are added, the seeding crystals have to be added prior to spontaneous crystallization occurs. The amount of seeding crystals may vary from 0.1 to 10 wt% in relation to the weight of the starting material 1-aminoindan. Preferably, 0.5 to 2wt% is used. The seeds can be added in a powder form or in a form of suspension in any type of liquid.

The formed or resolved diastereomeric salt obtained in steps (i), (ii) or (iii) can be isolated from the reaction mass by filtration. Moderately resolved diastereomeric salt may also be further purified using the first solvent system or the second solvent system, preferably the second solvent system.

In step (iv), the resolved diastereomeric salt is then treated with a base to obtain optically pure (R)-1-aminoindan as free base (neutralization). The base can be selected from alkali hydroxides, carbonates and hydrogen carbonates, preferably from potassium hydroxide, sodium hydroxide, sodium carbonate, potassium carbonate, sodium bicarbonate or potassium bicarbonate. Most preferably, sodium hydroxide is used.

The mother liquor obtained from step (ii) and/or step (iii) contains a diastereomeric salt of (S)-1-aminoindan or a diastereomeric salt of a mixture of (R)- and (S)- enantiomers of 1-aminoindan enriched in (S)-1-aminoindan with N-acetyl-L-glutamic acid. (S)-1-aminoindan or a mixture of (R)- and (S)- enatiomers of 1-aminoindan enriched in (S)-1-aminoindan can be obtained after neutralization of the salt recovered from the mother liquor after diastereomeric salts separation of 1-aminoindan with N-acetyl-L-glutamic acid. (S)-1-aminoindan or a mixture of (R)- and (S)-enatiomers of 1-aminoindan enriched in (S)-1-aminoindan can be racemized according to the processes known in the art such as by reacting said (S)-1-aminoindan with an alcoholate, e.g. tert-butoide, in an organic solvent, e.g. dimethylsulfoxide. Hence, the present invention also relates to a process for racemization of (S)-1-aminoindan which is recovered from the mother liquor after separating diastereomeric salts of 1-aminoindan and N-acetyl-L-glutamic acid.

The resolving agent N-acetyl-L-glutamic acid can be recovered from the aqueous mother liquor by usual known methods, as reported in literature, and reused for the same resolution.

A second aspect of the present invention is directed to a diastereomeric salt of 1-aminoindan with N-acetyl-L-glutamic acid. The salt may exist in racemic form, i.e. in form of a mixture of (R)-1-aminoindan N-acetyl-L-glutaminate and (S)-1-aminoindan N-acetyl-L-glutaminate, or in optically pure form, such as in form of optically pure (R)-1-aminoindan N-acetyl-L-glutaminate. Furthermore, the salt may exist in an acidic form having the chemical name 1-aminoindan N-acetyl-L-glutaminate (1:1), shown with the following structural formula: 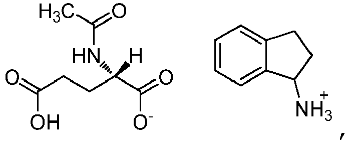 or in a neutral form, having the chemical name 1-aminoindan N-acetyl-L-glutaminate (2:1), shown with the following structural formula:

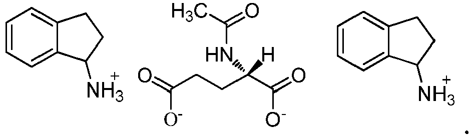

In case of an optically pure form of the salt, the acidic form is (R)-1-aminoindan N-acetyl-L-glutaminate (1:1), shown with the following structural formula: 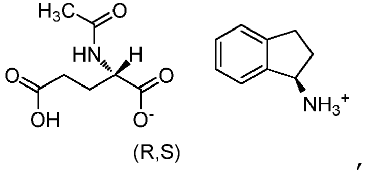 and the neutral form is (R)-1-aminoindan N-acetyl-L-glutaminate (2:1), shown with the following structural formula:

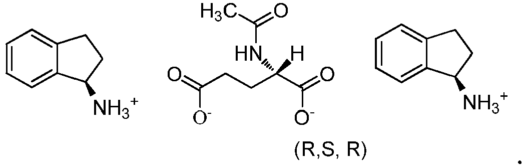

Racemic 1-aminoindan N-acetyl-L-glutaminate (1:1) may be characterized by the following NMR data: ¹H NMR (DMSO-d₆) δ: 7.64 (d, 1H), 7.53 (d, 1H), 7.18-7.34 (m, 3H), 4.58 (m, 1H), 4.07 (m, 1H), 2.94-3.06 (m, 1H), 2.74-2.90 (m, 1H), 2.34-2.48 (m, 1H), 2.10-2.28 (m, 2H), 1.86-2.00 (m, 1H), 1.71-1.86 (m, 5H); ¹³C NMR (DMSO-d₆) δ: 174.8, 174.1, 168.4, 143.6, 141.5, 128.3, 126.5, 124.7, 124.6, 54.9, 52.6, 31.9, 31.7, 29.8, 28.3, 22.7.

The racemic salt may be further characterized by a powder X-ray diffraction pattern with the following peaks (°2θ± 0.2) and relative intensity [%]:

| No. | Pos. [°2Theta] | Rel. Int. [%] |
|---|---|---|
| 1 | 7.2 | 15 |
| 2 | 13.7 | 68 |
| 3 | 14.4 | 24 |
| 4 | 15.4 | 23 |
| 5 | 17.9 | 27 |
| 6 | 21.6 | 51 |
| 7 | 23.0 | 100 |
| 8 | 23.7 | 31 |

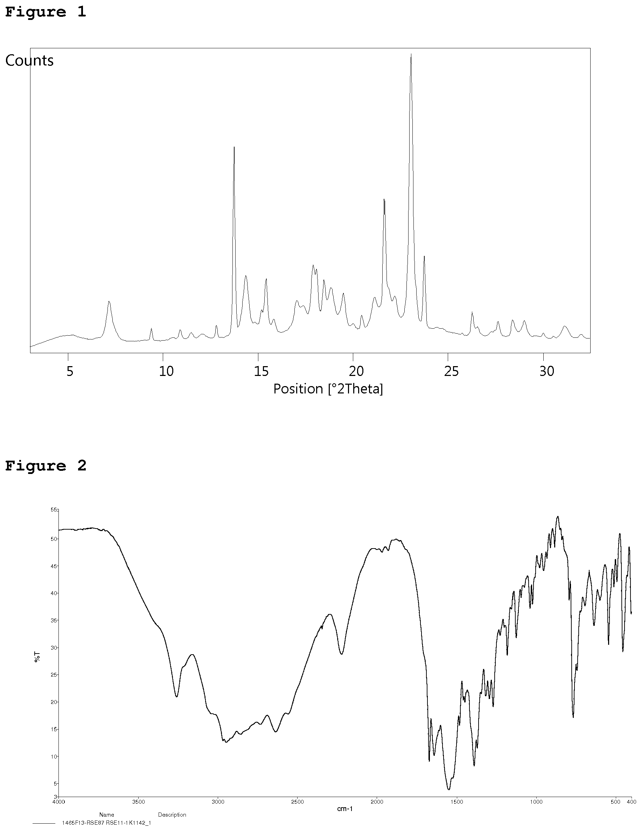

The racemic salt is in particular characterized by an XRPD spectrum as shown in Figure 1 and by an FT-IR as shown in Figure 2.

Optically pure (R)-1-aminoindan N-acetyl-L-glutaminate (2:1) may be characterized by the following NMR data: NMR: ¹H NMR (DMSO-d₆) δ: 7.61 (d, 1H), 7.38-7.47 (m, 2H), 7.13-7.31 (m, 6H), 4.42 (m, 2H), 4.11 (m, 1H), 2.89-2.99 (m, 2H), 2.77 (m, 2H), 2.34-2.44 (m, 2H), 2.28 (m, 1H), 2.13 (m, 1H), 1.70-1.88 (m, 6H), 1.58-1.69 (m, 1H); ¹³C NMR (DMSO-d₆) δ: 174.5, 173.6, 168.2, 144.2, 143.1, 127.6, 126.3, 124.5, 124.1, 55.8, 52.2, 33.8, 32.6, 29.7, 28.4, 22.7

The salt may be further characterized by a powder X-ray diffraction pattern with the following peaks (°2θ± 0.2) and relative intensity [%]:

| No. | Pos. [°2Theta] | Rel. Int. [%] |
|---|---|---|
| 1 | 7.4 | 53 |
| 2 | 14.1 | 68 |
| 3 | 15.2 | 81 |
| 4 | 17.4 | 41 |
| 5 | 19.9 | 26 |
| 6 | 21.5 | 100 |
| 7 | 22.9 | 32 |
| 8 | 24.6 | 26 |
| 9 | 28.5 | 21 |

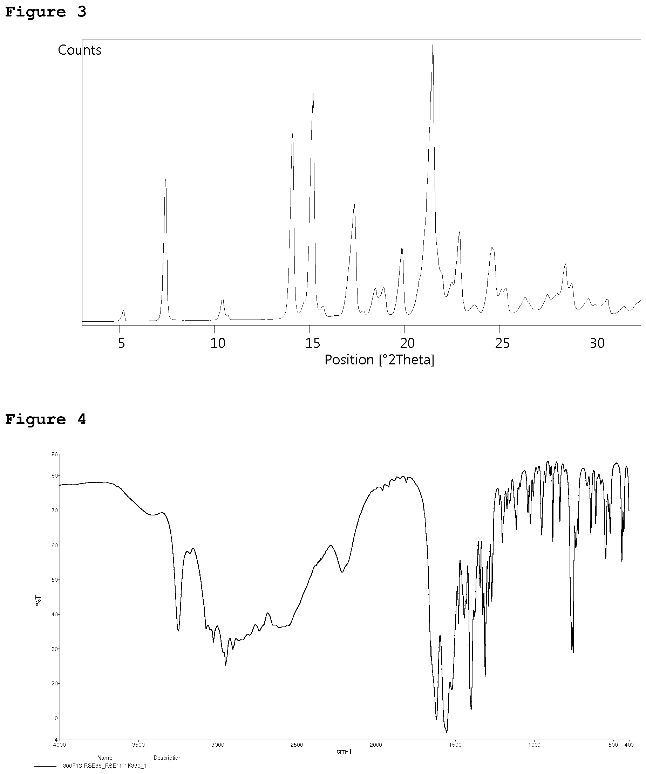

Optically pure (R)-1-aminoindan N-acetyl-L-glutaminate (2:1) is further characterized by an XRPD spectrum as shown in Figure 3 and by an FT-IR as shown in Figure 4.

The optically pure salt preferably exhibits optical purity of more than 90 area %, preferably more than 95 area %, most preferably more than 99 area %. Optical purity is determined by means of HPLC method.

The salts of 1-aminoindan with N-acetyl-L-glutamic acid according to the present invention may be used in the process for preparation of rasagiline and/or acid addition salt thereof according to any known procedure such as disclosed in e.g. EP 812190, WO 2012/153349, WO 2009/141737, EP 2364967 or WO 2011/087791. Rasagilin acid addition salt may be selected from rasagiline mesylate, esylate, sulfate, (hemi)-L-tartrate, which were disclosed in WO 95/11016, rasagiline hydrochloride disclosed in EP 436492, tannate salt of rasagiline disclosed in WO 2008/076315, acid addition salts of rasagiline with organic diacids, specifically rasagiline edisylate and rasagiline oxalate disclosed in WO 2008/019871, rasagiline fumarate salt, succinate salt, besylate salt etc disclosed in WO 2010/007181, citrate salt disclosed in WO 2010/085354.

Accordingly, the present invention also relates to a process for preparing optically pure (R)-1-aminoindan according to the first aspect of the invention, wherein said optically pure (R)-1-aminoindan is used for the preparation of rasagiline or a pharmaceutically acceptable salt thereof.

Moreover, the present invention also relates to a process for preparing rasagiline or a pharmaceutically acceptable salt thereof comprising the preparation of optically pure (R)-1-aminoindan according to the first aspect of the invention and a further step in which said optically pure (R)-1-aminoindan is converted into rasagiline or a pharmaceutically acceptable salt thereof. The conversion of the optically pure (R)-1-aminoindan into rasagiline or a pharmaceutically acceptable salt thereof can be performed by processes known in the art such as processes described in EP 436492, WO 2009/14730 or WO 2012/116752.

The present invention is further described in greater detail as illustrated in the following of examples.

### Examples

### Methods

**X-ray powder diffraction patterns** were obtained by a PANalytical PW3040/60 X'Pert PRO diffractometer using CuKα radiation of 1.541874 Å.

**The FT-IR spectra** were obtained with a FT-IR spectrometer Perkin Elmer Frontier at a resolution of 4 cm⁻¹ from 4000 cm⁻¹ to 400 cm⁻¹. The samples of Form K were prepared as KBr discs.

### Chromatographic purity, enatiomeric purity and assay were determined by HPLC method:

The product's purity was assessed by high pressure liquid chromatography (HPLC) using a octadecyl silica column (type YMC Triart C-18 150 x 4.6 mm i.d., 3 µm particles); column temperature: 25°C; detector: UV 210 nm; flow rate: 1.0 ml/min; injection volume: 2 µl; mobile phase A: 0.01M phosphate buffer solution pH 8; mobile phase B: 90% acetonitrile; Gradient: 0'=2%B, 10'=30%B, 29'-33'=100%B, 37'-42'=2%B. This HPLC method is used for the analysis of **chromatographic purity and assay.**

**Enantiomeric purity** was assessed by HPLC method using a polysaccharide based chiral column (type Chiralpak AD-H, 250 x 4.6 mm, 5 µm particles). Mobile phase (which was a mixture of hexane, ethanol and butylamine in the ratio 980:20:0.4 (V/V/V); flow-rate: 0.7 ml/min; UV detector: 265 nm; injection volume: 10 µl) was used for separation.

### Sample preparation:

Assay and chromatographic purity: The sample solution was prepared in concentration of about 1 - 1.5 mg/ml. Dilution solvent was acetonitrile and water.

Enantiomeric purity: The sample solution was prepared in concentration of about 10 mg/ml. Dilution solvent was a mixture of ethanol and 2-propanol.

### Calculation:

Chromatographic purity: Area percent method was used. Solvent peaks were disregarded.

Assay: External standard method was used.

Enantiomeric purity: Area percent method was used.

### Example 1

### Preparation of 1-indanone oxime

A reactor was charged with 200 g of 1-indanone (1.5133 mol) and 1440 ml of 96 % ethanol. The mixture was stirred at room temperature (20° - 25°C) for 15 minutes. To the mixture 189.3 g of hydroxylamine hydrochloride (2.7239 mol) was added and the resulting mixture was stirred for 10 minutes. A thick suspension was formed to which 1040 ml of 2N NaOH were added, the suspension was stirred at room temperature for 1 hour. The solvent was evaporated under reduced pressure to obtain a solid residue. To the residue 2400 ml of water and 2500 ml of ethyl acetate were added and stirred for 15 minutes. The phases were separated and the aqueous phase was washed with 2500 ml of ethyl acetate. The phases were separated. Both organic phases were combined and evaporated under reduced pressure at temperatures below 40 °C. 204 g of the titled product was obtained in a molar yield of 91.6%.

### Example 2

### Preparation of racemic 1-aminoindan

A reactor was charged at 25°C with 585.8 g of 1-indanone oxime and 4960 ml of 4.2 M ammonia in methanol, then to the reactor 117 g Catalyst Raney Ni (in the form of suspension in methanol) was added and the mixture was hydrogenated under stirring at constant pressure of 3.5 ± 0.1 bar. The hydrogenation reaction was carried out at a temperature of 42 ± 2°C for 20 hours. After completion of the reaction, the reaction mixture was cooled to 25 ± 2°C, the suspension was filtered to separate the catalyst. The filtrate was concentrated under reduced pressure and at a temperature of less than 40 °C to constant weight to obtain 558.0g of an oily product. Assay: 95.1% HPLC, molar yield wass 100%. The product was kept in an inert atmosphere, at temperatures below 10°C and protected from light.

### Example 3: Preparation of diastereomeric salt (R,S)-1-aminoindan N-acetyl-L-glutaminate (1:1)

### Example 3a

42.9 g of (R,S)-1-aminoindan, 42.9 ml of ethanol and 174 ml of TBME (tert-butyl methyl ether) were added to a reactor. To this mixture 42.6 g of N-acetyl-L-glutamic acid were added under stirring at room temperature. The ratio of (R,S)-1-aminoindan and N-acetyl-L-glutamic acid was 1:0.7. After a few minutes, the product started to crystallize from the solution and a thick suspension was formed to which 87 ml of TBME were added to obtain a miscible suspension, which was further stirred for 30 minutes at room temperature. After that the suspension was cooled to the final crystallization temperature of 5 °C and left at this temperature under stirring for 2 hours. The product was filtered off using vacuum filtration and washed with 43 ml of TBME.

Weight: 89.5 g
Molar yield: 68 %
Chromatographic purity: 96.5 area %
Enantiomeric purity: 50.03 area %
Content (HPLC assay): 78.8 %
NMR:
¹H NMR (DMSO-d₆) δ: 7.64 (d, 1H), 7.53 (d, 1H), 7.18-7.34 (m, 3H), 4.58 (m, 1H), 4.07 (m, 1H), 2.94-3.06 (m, 1H), 2.74-2.90 (m, 1H), 2.34-2.48 (m, 1H), 2.10-2.28 (m, 2H), 1.86-2.00 (m, 1H), 1.71-1.86 (m, 5H)
¹³C NMR (DMSO-d₆) δ: 174.8, 174.1, 168.4, 143.6, 141.5, 128.3, 126.5, 124.7, 124.6, 54.9, 52.6, 31.9, 31.7, 29.8, 28.3, 22.7

### Example 3b

10 g of (R,S)-1-aminoindan, 10 ml of ethanol and 40 ml of TBME were added to a reactor. To this mixture 14.2 g of N-acetyl-L-glutamic acid were added under stirring at room temperature. The ratio of (R,S)-1-aminoindan and N-acetyl-L-glutamic acid was 1:1. After a few minutes, the product started to crystallize from the solution and a thick suspension was formed to which 20 ml of TBME were added to obtain a miscible suspension, which was further stirred for 30 minutes at room temperature. After that the suspension was cooled to the final crystallization temperature of 5°C and left at this temperature under stirring for 2 hours. The product was filtered off using vacuum filtration and washed with 10 ml of TBME.

Weight: 25.3 g
Molar yield: 73 %
Chromatographic purity: 96.1 area %
Enantiomeric purity: 50.0 area %
Content: 70.0 %

### Example 4

### Optical resolution to obtain (R)-1-aminoindan N-acetyl-L-glutaminate

In a reactor 70 g of (R,S)-1-aminoindan N-acetyl-L-glutaminate and 2100 ml of ethanol were added. Over the next 45 minutes the crystallization mixture was heated under stirring to a temperature of 79°C. After reaching 79°C, the mixture was stirred at this temperature for 5 minutes, then the mixture was cooled within 45 minutes to a temperature of 40°C. At this temperature the mixture was seeded with 0.7 g of crystalline (R)-1-aminoindan N-acetyl-L-glutaminate. The mixture was stirred at this temperature for 20 minutes, and then the suspension was cooled within 30 minutes to the final crystallization temperature of 5°C and left at this temperature under stirring overnight. The product was isolated using vacuum filtration and washed with 30 ml of cold ethanol.

Weight: 29.9g
Molar yield: 42.6%
Chromatographic purity: 99.97% area
Enantiomeric purity: 92.3% area

### Example 5

### Recrystallization of (R)-1-aminoindan N-acetyl-L-glutaminate (2:1)

In a reactor 24 g of (R)-1-aminoindan N-acetyl-L-glutaminate and 1440ml of ethanol were added. Over the next 45 minutes the crystallization mixture was heated under stirring to a temperature of 79°C. After reaching 79°C, 14.4 ml of water was added to the mixture and stirred at this temperature for 60 minutes. After that 240 ml of ethanol was added and the mixture was cooled within 45 minutes to a temperature of 40°C. At this temperature the mixture was seeded with 0.24 g of crystalline (R)-1-aminoindan N-acetyl-L-glutaminate. The mixture was stirred at this temperature for 20 minutes, and then the suspension was cooled within 30 minutes to the final crystallization temperature of 5°C and left at this temperature under stirring overnight. The product was isolated using vacuum filtration and washed with 20 ml of cold ethanol.

Weight: 24.0 g
Molar yield: 95 %
Chromatographic purity: 100 % area
Enantiomeric purity: 99.1 area %
NMR: ¹H NMR (DMSO-d₆) δ: 7.61 (d, 1H), 7.38-7.47 (m, 2H), 7.13-7.31 (m, 6H), 4.42 (m, 2H), 4.11 (m, 1H), 2.89-2.99 (m, 2H), 2.77 (m, 2H), 2.34-2.44 (m, 2H), 2.28 (m, 1H), 2.13 (m, 1H), 1.70-1.88 (m, 6H), 1.58-1.69 (m, 1H); ¹³C NMR (DMSO-d₆) δ: 174.5, 173.6, 168.2, 144.2, 143.1, 127.6, 126.3, 124.5, 124.1, 55.8, 52.2, 33.8, 32.6, 29.7, 28.4, 22.7; Elemental analysis: Calculated: C 65.91, H 7.30, N 9.22; Found: C 65.71, H 7.66, N 9.15

### Example 6

### Optical resolution to obtain (R)-1-aminoindan N-acetyl-L-glutaminate

In a reactor 2.67 g of (R,S)-1-aminoindan N-acetyl-L-glutaminate and 80 ml of ethanol were added. Over the next 45 minutes the crystallization mixture was heated under stirring to a temperature of 79°C. After reaching 79°C, the mixture was stirred at this temperature for 15 minutes, then the mixture was cooled within 45 minutes to a temperature of 5°C. The mixture was left at this temperature under stirring overnight. The precipitated product was isolated using vacuum filtration.

Weight: 1.21 g
Molar yield: 45 %
Chromatographic purity: 99.9 area %
Enantiomeric purity: 90.88 area %

### Example 6a

### Recrystallization of (R)-1-aminoindan N-acetyl-L-glutaminate

In a reactor 1 g of (R)-1-aminoindan N-acetyl-L-glutaminate obtained in Example 6 and 70 ml of ethanol were added. Over the next 45 minutes the crystallization mixture was heated under stirring to a temperature of 79°C. After reaching 79°C, 0.7 ml of water was added to the mixture and stirred at this temperature for 15 minutes. The mixture was cooled within 45 minutes to the final crystallization temperature of 5°C and left at this temperature under stirring overnight. The precipitated product was isolated using vacuum filtration.

Weight: 0.83 g
Molar yield: 83 %
Chromatographic purity: 100 area %
Enantiomeric purity: 98.72 area %

### Example 7

### Optical resolution to obtain (R)-1-aminoindan N-acetyl-L-glutaminate

In a reactor 2.67 g of (R,S)-1-aminoindan N-acetyl-L-glutaminate and 80 ml of ethanol were added. Over the next 45 minutes the crystallization mixture was heated under stirring to a temperature of 79°C. After reaching 79°C, the mixture was stirred at this temperature for 15 minutes, then the mixture was cooled within 25 minutes to a temperature of 40°C. At this temperature the mixture was seeded with 0.03 g of crystalline (R)-1-aminoindan N-acetyl-L-glutaminate. The mixture was stirred at the same temperature for 20 minutes, and then the suspension was cooled within 20 minutes to the final crystallization temperature of 5°C and left at this temperature under stirring overnight. The precipitated product was isolated using vacuum filtration.

Weight: 1.23g
Molar yield: 46%
Chromatographic purity: 99.9% area
Enantiomeric purity: 92.56% area

### Example 7a

### Recrystallization of (R)-1-aminoindan N-acetyl-L-glutaminate

In a reactor 1 g of (R)-1-aminoindan N-acetyl-L-glutaminate obtained in Example 7 and 70 ml of ethanol were added. Over the next 45 minutes the crystallization mixture was heated under stirring to a temperature of 79°C. After reaching 79°C, 0.7 ml of water was added to the mixture and stirred at this temperature for 15 minutes. After that the mixture was cooled within 25 minutes to a temperature of 40°C. At this temperature the mixture was seeded with 0.01 g of crystalline (R)-1-aminoindan N-acetyl-L-glutaminate. The mixture was stirred at the same temperature for 20 minutes, then the suspension was cooled within 20 minutes to the final crystallization temperature of 5°C and left at this temperature under stirring overnight. The precipitated product was isolated using vacuum filtration.

Weight: 0.88 g
Molar yield: 88 %
Chromatographic purity: 100 % area
Enantiomeric purity: 99.19 area %

### Example 8

### Optical resolution to obtain (R)-1-aminoindan N-acetyl-L-glutaminate

In a reactor 2.67 g of (R,S)-1-aminoindan N-acetyl-L-glutaminate and 80 ml of ethanol were added. Over the next 45 minutes the crystallization mixture was heated under stirring to a temperature of 79°C. After reaching 79°C, the mixture was stirred at this temperature for 15 minutes, then the mixture was cooled within 25 minutes to a temperature of 45°C. At this temperature the mixture was seeded with 0.03 g of crystalline (R)-1-aminoindan N-acetyl-L-glutaminate. The mixture was stirred at the same temperature for 20 minutes, and then the suspension was cooled within 25 minutes to the final crystallization temperature of 5°C and left at this temperature under stirring overnight. The precipitated product was isolated using vacuum filtration.

Weight: 1.20g
Molar yield: 45%
Chromatographic purity: 99.9% area
Enantiomeric purity: 92.38% area

### Example 8a

### Recrystallization of (R)-1-aminoindan N-acetyl-L-glutaminate

In a reactor 1 g of (R)-1-aminoindan N-acetyl-L-glutaminate obtained in Example 8 and 70 ml of ethanol were added. Over the next 45 minutes the crystallization mixture was heated under stirring to a temperature of 79°C. After reaching 79°C, 0.7 ml of water was added to the mixture and stirred at this temperature for 15 minutes. After that the mixture was cooled within 20 minutes to a temperature of 45°C. At this temperature the mixture was seeded with 0.01 g of crystalline (R)-1-aminoindan N-acetyl-L-glutaminate. The mixture was stirred at the same temperature for 20 minutes, then the suspension was cooled within 25 minutes to the final crystallization temperature of 5°C and left at this temperature under stirring overnight. The precipitated product was isolated using vacuum filtration.

Weight. 0.86 g
Molar yield: 86 %
Chromatographic purity: 100 % area
Enantiomeric purity: 99.05% area

### Example 9

### Optical resolution to obtain (R)-1-aminoindan N-acetyl-L-glutaminate

In a reactor 2.67 g of(R,S)-1-aminoindan N-acetyl-L-glutaminate and 80 ml of ethanol was added. Over the next 45 minutes the crystallization mixture was heated under stirring to a temperature of 79°C. After reaching 79°C, the mixture was stirred at this temperature for 15 minutes, then the mixture was cooled within 25 minutes to a temperature of 35°C. At this temperature the mixture was seeded with 0.03 g of crystalline (R)-1-aminoindan N-acetyl-L-glutaminate. The mixture was stirred at the same temperature for 20 minutes, and then the suspension was cooled within 20 minutes to the final crystallization temperature of 5°C and left at this temperature under stirring overnight. The precipitated product was isolated using vacuum filtration.

Weight: 1.20g
Molar yield: 45%
Chromatographic purity: 99.9% area
Enantiomeric purity: 92.50% area

### Example 9a

### Recrystallization of (R)-1-aminoindan N-acetyl-L-glutaminate

In a reactor 1 g of (R)-1-aminoindan N-acetyl-L-glutaminate obtained in Example 9 and 70 ml of ethanol were added. Over the next 45 minutes the crystallization mixture was heated under stirring to a temperature of 79°C. After reaching 79°C, 0.7 ml of water was added to the mixture and stirred at this temperature for 15 minutes. After that the mixture was cooled within 25 minutes to a temperature 35°C. At this temperature the mixture was seeded with 0.01 g of crystalline (R)-1-aminoindan N-acetyl-L-glutaminate. The mixture was stirred at the same temperature for 20 minutes, then the suspension was cooled within 20 minutes to the final crystallization temperature of 5°C and left at this temperature under stirring overnight. The precipitated product was isolated using vacuum filtration.

Weight = 0.89 g
Molar yield = 89 %
Chromatographic purity = 100 % area
Enantiomeric purity=99.05% area

### Example 10

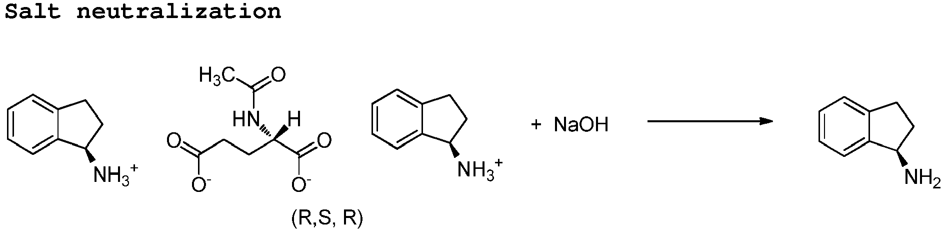

20 g of (R)-1-aminoindan N-acetyl-L-glutaminate (2:1), 200 ml of 1 M NaOH and 200 ml ethyl acetate were charged to a reactor. The mixture was stirred for 0.5 h at room temperature, after that the phases were separated in a separating funnel. The water phase was washed with ethyl acetate; the organic phases were collected and concentrated under the reduced pressure to the constant weight to obtain 11.84 g of the oily product (R)-1-aminoindan.

Molar yield: 98.8 %
Chromatographic purity: 99.83 %area
Enantiomeric purity: 99.36 %area

### Example 11

### Preparation of rasagiline

To a reactor 8.14 g of propargyl benzenesulphonate, 75.71 g of (R)-1-aminoindan hydrochloride and 57 ml of toluene were added. The mixture was heated to 30°C, and then 18 ml of 15% NaOH were slowly added, the mixture was further stirred at the same temperature for 29 hours.

After completing the reaction 38.5 ml of water and 16 ml of toluene were added to the reaction mixture and stirred for 15 minutes, then the stirring was stopped and the phases were separated. The organic phase was washed with 38.5 ml of water and stirred for 15 minutes, then the phases were separated. The upper organic phase was washed with 38.5 ml of 10% NaOH and stirred for 15 minutes, than the phases were separated. The organic phase was mixed with 23 ml of water and the pH was adjusted to 3.2 by addition of 12 ml of 10% solution of H₂SO₄. The phases were separated and the organic phase was discarded. To the water phase 38.5 ml of toluene were added and the pH was adjusted to 7.3 by adding 9 ml of 10% NaOH. The phases were separated and the water phase was extracted twice by addition of 38.5 ml of toluene and adjusting pH to 7.3 by addition of 10% solution of NaOH. Organic phases were collected and the solvent of the organic phase was evaporated under vacuum by heating and stirring. After the evaporation 4.87 g of an oily product with an assay of 74.9% was obtained.
Yield: 65.8%,
Enatiomeric purity: 99.96 area %

### Example 12

### Preparation of rasagiline mesylate

3.23 g of the oily product obtained according to Example 11 were mixed with 22 ml of 2-propanol and the mixture was heated to 80°C. At this temperature methanesulfonic acid (MSA, 1.47 g) was added slowly to the batch. The resulting mixture was cooled to 60°C and seeded with 1% of seeding crystals and stirred at a temperature of 55°C for 60 minutes. Then the suspension was cooled with the cooling at a rate of 0.5°C/min to a temperature of 10°C. The mixture was stirred at this temperature for 1 hour, then the product was filtered and washed with 3 ml of 2-propanol. The product was dried and 2.90 g of crystalline product was obtained (assay 99.93%, molar yield 76.6%, enatiomeric purity 100.00 area %, chromatographic purity 100.00%).

### Example 13

### Preparation of rasagiline hemitartrate

In 726 ml of 2-propanol 40.9 g of L(+)-tartaric acid (anhydrous) were dissolved under heating to 40°C to obtain Solution 1. Separately Solution 2 was prepared by dissolving 110.3 g of rasagiline free base in 168 ml of 2-propanol under stirring and heating to 40°C. Solution 2 was slowly added under rigorous stirring to Solution 1 within 130 minutes. The obtained suspension was then cooled to 15-20°C within 1 hour and further stirred at this temperature for 1 hour. The product was filtered, washed with 70 ml of pre-cooled 2-propanol (having a temperature of 5-10°C) and dried in vacuum drier at 50°C and a pressure under 50 mbar. 132.6 of crystalline product was obtained with an assay of 100% and a molar yield of 88.0% (enatiomeric purity 100.00 area%, chromatographic purity 100.00 area%).

### Example 14

### Preparation of R-aminoindan hydrochloride

To a reactor 22.3 g of R-aminoindan and 68 ml of 2-propanol were charged at a temperature of 10°C under stirring and under inert atmosphere. Then 83 ml of 5-6 N HCl in 2-propanol were added slowly while the temperature was maintained under 20°C, preferably at about 10°C. After the addition of HCl was completed the mixture was cooled to 5°C and stirred at this temperature for 30 minutes. Then the product was filtered, washed with 22 ml of 2-propanol and dried in a vacuum drier at 40°C and reduced pressure (< 100 mbar) until LoD < 1.

21.8 g of the product with an assay of 99,6 % and a molar yield of 84,5 % were obtained.

### Example 15

### Preparation of propargyl benzensulfonate

To a reactor 525 ml of TBME, 15.6 ml of prop-2-yn-1-ol (15 g, 0.2675 mol) and 37.6 ml of benzenesulfonyl chloride (52 g, 0,2943 mol) were charged at room temperature. The mixture was cooled to - 10°C within 60 minutes under stirring. At this temperature 75.05 g of powdered KOH were added within 30 minutes. During the addition of KOH the temperature was maintained between -10°C and 0°C. Then 300 ml of pre-cooled water having a temperature of 0° C were added slowly during 15 minutes, the obtained suspension was then stirred for 2 hours at -10°C. Then the mixture was heated to room temperature within 60 minutes and 1 g of active charcoal was added followed by 30 minutes of stirring. The mixture was filtered using Randalite filter, the two phases of the filtrate were separated, and to the organic phase alkaline water (10 g KOH in 100 ml of water) was added and stirred for 15 minutes. The phases were separated and the organic phase was evaporated at a temperature below 40°C and under reduced pressure to obtain 47.5 g of an oily product with an assay of 100% (yield 91%).

## Claims

1. A process for preparation of optically pure (R)-1-aminoindan by a diastereomeric resolution of 1-aminoindan using N-acetyl-L-glutamic acid as a resolving agent.

2. The process according to claim 1, which comprises the following steps:
(i) forming diastereomeric salts of 1-aminoindan with N-acetyl-L-glutamic acid,
(ii) separating the diastereomeric salts in a first solvent system at a specified temperature range by means of fractional crystallization,
(iii) optionally, crystallizing the diastereomeric salt obtained in step (ii) in a second solvent system to get desired optical purity,
(iv) neutralizing the diastereomeric salt obtained in step (ii) or step (iii) to obtain optically pure (R)-1-aminoindan.

3. The process according to claim 2, wherein the diastereomeric salt formation in step (i) as well as resolution in steps (ii) and optionally (iii) are carried out in the same solvent system or in a different solvent system.

4. The process according to claim 2 or 3, wherein the solvent system used in the resolution steps (ii) and/or (iii) is a combination of alcoholic solvents such as methanol, ethanol, 1-propanol and 2-propanol with 0-10% (v/v) of dipolar solvents such as N,N-dimethylformamide, N,N-dimethylacetamide, N-methyl-2-pyrrolidone, dimethylsulfoxide or water.

5. The process according to any of the claims 2 to 4, wherein the solvent system used in steps (ii) and (iii) comprises ethanol with 0-10% (v/v) of water.

6. The process according to any of the claims 2 to 5, wherein the first solvent system comprises ethanol and the second solvent system comprises a mixture of ethanol with 0.1-3% (v/v) of water, preferably the second solvent system comprises a mixture of ethanol with 0.5-2% (v/v) of water, more preferably 1% (v/v) of water.

7. The process according to any of the claims 2 to 6, wherein the crystallization in steps (ii) and/or (iii) is initiated by addition of seeding crystals of diastereomeric salt of R-1-aminoindan and N-acetyl-L-glutamic acid, wherein addition of the seeding crystals is preferably carried out at a temperature of 20°C to 65°C, more preferably at 30°C to 50°C, most preferably at 35°C to 45°C.

8. A diastereomeric salt of 1-aminoindan with N-acetyl-L-glutamic acid, which preferably exists in racemic form or in optically pure form.

9. A diastereomeric salt according to claim 8, wherein the salt exists in an acidic form having chemical name 1-aminoindan N-acetyl-L-glutaminate (1:1) or in a neutral form, having chemical name 1-aminoindan N-acetyl-L-glutaminate (2:1).

10. (R)-1-aminoindan N-acetyl-L-glutaminate (1:1), **characterized by** the following structural formula:

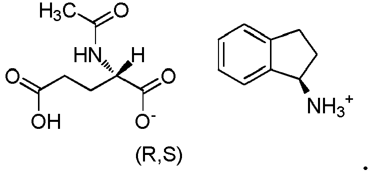

11. (R)-1-aminoindan N-acetyl-L-glutaminate (2:1), **characterized by** the following structural formula:

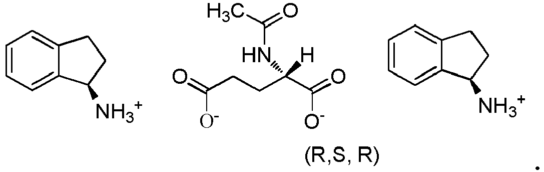

12. Use of the compounds according to any of the claims 8 to 11 for the preparation of rasagiline and/or acid addition salt thereof.

13. Use of N-acetyl-L-glutamic acid in the preparation of optically pure (R)-1-aminoindan.

14. Process for preparing optically pure (R)-1-aminoindan according to any one of claims 1 to 7, wherein said optically pure (R)-1-aminoindan is used to prepare rasagiline or a pharmaceutically acceptable salt thereof.

15. Process for preparing rasagiline or a pharmaceutically acceptable salt thereof comprising the preparation of optically pure (R)-1-aminoindan according to the process of any one of claims 1 to 7 and a further step in which said optically pure (R)-1-aminoindan is converted into rasagiline or a pharmaceutically acceptable salt thereof.

## Patentansprüche

1. Verfahren zur Herstellung von optisch reinem (R)-1-Aminoindan durch eine Diastereomerentrennung von 1-Aminoindan unter Verwendung von N-Acetyl-L-glutaminsäure als ein Trennungsmittel.

2. Verfahren nach Anspruch 1, das die folgenden Schritte umfasst:
(i) Bilden von diastereomeren Salzen von 1-Aminoindan mit N-Acetyl-L-glutaminsäure,
(ii) Trennen der diastereomeren Salze in einem ersten Lösungsmittelsystem in einem vorgegebenen Temperaturbereich mittels fraktionierter Kristallisation,
(iii)gegebenenfalls Kristallisieren des in Schritt (ii) erhaltenen diastereomeren Salzes in einem zweiten Lösungsmittelsystem, um die gewünschte optische Reinheit zu erhalten,
(iv) Neutralisieren des in Schritt (ii) oder Schritt (iii) erhaltenen diastereomeren Salzes, um optisch reines (R)-1-Aminoindan zu erhalten.

3. Verfahren nach Anspruch 2, bei dem die Bildung des diastereomeren Salzes in Schritt (i) sowie die Trennung in den Schritten (ii) und gegebenenfalls (iii) im gleichen Lösungsmittelsystem oder in einem unterschiedlichen Lösungsmittelsystem durchgeführt werden.

4. Verfahren nach Anspruch 2 oder 3, bei dem das in den Trennungsschritten (ii) und/oder (iii) verwendete Lösungsmittelsystem eine Kombination von alkoholischen Lösungsmitteln, wie beispielsweise Methanol, Ethanol, 1-Propanol und 2-Propanol, mit 0 bis 10 Vol.-% dipolaren Lösungsmitteln, wie beispielsweise N,N-Dimethylformamid, N,N-Dimethylacetamid, N-Methyl-2-pyrrolidon, Dimethylsulfoxid oder Wasser, ist.

5. Verfahren nach einem der Ansprüche 2 bis 4, bei dem das in den Schritten (ii) und (iii) verwendete Lösungsmittelsystem Ethanol mit 0 bis 10 Vol.-% Wasser enthält.

6. Verfahren nach einem der Ansprüche 2 bis 5, bei dem das erste Lösungsmittelsystem Ethanol enthält und das zweite Lösungsmittelsystem eine Mischung von Ethanol mit 0,1 bis 3 Vol.-% Wasser enthält, wobei vorzugsweise das zweite Lösungsmittelsystem eine Mischung von Ethanol mit 0,5 bis 2 Vol.-% Wasser, insbesondere 1 Vol.-% Wasser, enthält.

7. Verfahren nach einem der Ansprüche 2 bis 6, bei dem die Kristallisation in den Schritten (ii) und/oder (iii) durch Zugabe von Impfkristallen von diastereomerem Salz von R-1-Aminoindan und N-Acetyl-L-glutaminsäure initiiert wird, wobei die Zugabe der Impfkristalle vorzugsweise bei einer Temperatur von 20°C bis 65°C, insbesondere 30°C bis 50°C, besonders bevorzugt 35°C bis 45°C, durchgeführt wird.

8. Diastereomeres Salz von 1-Aminoindan mit N-Acetyl-L-glutaminsäure, das vorzugsweise in racemischer Form oder in optisch reiner Form vorliegt.

9. Diastereomeres Salz nach Anspruch 8, bei dem das Salz in einer sauren Form mit dem chemischen Namen 1-Aminoindan-N-acetyl-L-glutamat (1:1) oder in einer neutralen Form mit dem chemischen Namen 1-Aminoindan-N-acetyl-L-glutamat (2:1) vorliegt.

10. (R)-1-Aminoindan-N-acetyl-L-glutamat (1:1), das durch die folgende Strukturformel gekennzeichnet ist:

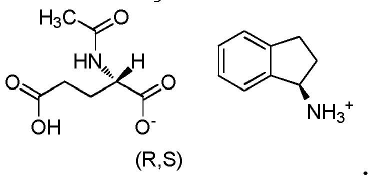

11. (R)-1-Aminoindan-N-acetyl-L-glutamat (2:1), das durch die folgende Strukturformel gekennzeichnet ist:

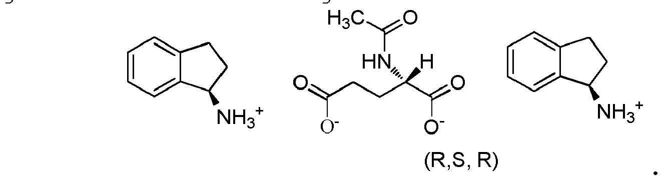

12. Verwendung der Verbindungen nach einem der Ansprüche 8 bis 11 zur Herstellung von Rasagilin und/oder Säureadditionssalzen davon.

13. Verwendung von N-Acetyl-L-glutaminsäure bei der Herstellung von optisch reinem (R)-1-Aminoindan.

14. Verfahren zur Herstellung von optisch reinem (R)-1-Aminoindan nach einem der Ansprüche 1 bis 7, bei dem das optisch reine (R)-1-Aminoindan verwendet wird, um Rasagilin oder ein pharmazeutisch annehmbares Salz davon herzustellen.

15. Verfahren zur Herstellung von Rasagilin oder eines pharmazeutisch annehmbaren Salzes davon, welches die Herstellung von optisch reinem (R)-1-Aminoindan gemäß dem Verfahren nach einem der Ansprüche 1 bis 7 und einen weiteren Schritt, bei dem das optisch reine (R)-1-Aminoindan zu Rasagilin oder einem pharmazeutisch annehmbaren Salz davon umgewandelt wird, umfasst.

## Revendications

1. Procédé pour la préparation de (R)-1-aminoindane optiquement pur par une résolution de diastéréoisomères de 1-aminoindane à l'aide d'acide N-acétyl-L-glutamique en tant que solvant de résolution.

2. Procédé selon la revendication 1, qui comprend les étapes suivantes consistant à :
(i) former des sels diastéréoisomères de 1-aminoindane avec de l'acide N-acétyl-L-glutamique,
(ii) séparer au moyen d'une cristallisation fractionnée les sels diastéréoisomères dans un premier système de solvant dans une plage de température spécifiée,
(iii) en option, faire cristalliser dans un second système de solvant le sel diastéréoisomère obtenu dans l'étape (ii), pour atteindre la pureté optique désirée,
(iv) neutraliser le sel diastéréoisomère obtenu dans l'étape (ii) ou l'étape (iii), pour obtenir du (R)-1-aminoindane optiquement pur.

3. Procédé selon la revendication 2, dans lequel la formation de sels diastéréoisomères dans l'étape (i) ainsi que la résolution dans l'étape (ii) et en option l'étape (iii) sont effectuées dans le même système de solvant ou dans des systèmes de solvants différents.

4. Procédé selon la revendication 2 ou 3, dans lequel le système de solvant utilisé dans l'étape de résolution (ii) et/ou l'étape de résolution (iii) est une association de solvants alcooliques tels que le méthanol, l'éthanol, le 1-propanol et le 2-propanol avec 0-10 % (v/v) de solvants dipolaires tels que le N,N-diméthylformamide, le N,N-diméthylacétamide, la N-méthyl-2-pyrrolidone, le diméthylsulfoxyde ou l'eau.

5. Procédé selon l'une quelconque des revendication 2 à 4, dans lequel le système de solvant utilisé dans les étapes (ii) et (iii) comprend de l'éthanol avec 0-10 % (v/v) d'eau.

6. Procédé selon l'une quelconque des revendication 2 à 5, dans lequel le premier système de solvant comprend de l'éthanol et le second système de solvant comprend un mélange d'éthanol avec 0,1-3 % (v/v) d'eau, de préférence le second système de solvant comprend un mélange d'éthanol avec 0,5-2 % (v/v) d'eau, de façon plus particulièrement préférée 1 % (v/v) d'eau.

7. Procédé selon l'une quelconque des revendication 2 à 6, dans lequel la cristallisation dans l'étape (ii) et/ou l'étape (iii) est déclenchée par addition de cristaux germes de sel diastéréoisomère de R-1-aminoindane et acide N-acétyl-L-glutamique, l'addition des cristaux germes étant effectuée de préférence à une température de 20 °C à 65 °C, de façon plus particulièrement préférée de 30 °C à 50 °C, de façon tout particulièrement préférée de 35 °C à 45 °C.

8. Sel diastéréoisomère de 1-aminoindane avec l'acide N-acétyl-L-glutamique, qui se trouve de préférence sous forme racémique ou sous forme optiquement pure.

9. Sel diastéréoisomère selon la revendication 8, où le sel se trouve sous une forme acide portant la dénomination chimique N-acétyl-L-glutamate de 1-aminoindane (1:1) ou sous une forme neutre, portant la dénomination chimique N-acétyl-L-glutamate de 1-aminoindane (2:1).

10. N-acétyl-L-glutamate de (R)-1-aminoindane (1:1), **caractérisé par** la formule structurale suivante :

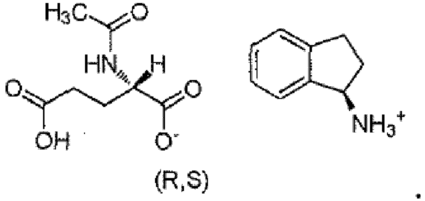

11. N-acétyl-L-glutamate de (R)-1-aminoindane (2:1), **caractérisé par** la formule structurale suivante :

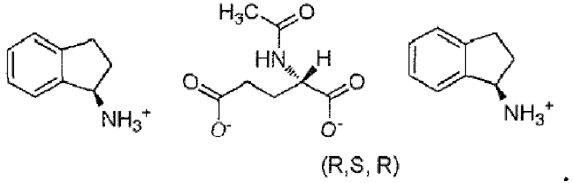

12. Utilisation des composés selon l'une quelconque des revendications 8 à 11 pour la préparation de rasagiline et/ou d'un sel d'addition avec un acide de celle-ci.

13. Utilisation d'acide N-acétyl-L-glutamique dans la préparation de (R)-1-aminoindane optiquement pur.

14. Procédé pour la préparation de (R)-1-aminoindane optiquement pur selon l'une quelconque des revendications 1 à 7, dans lequel on utilise ledit (R)-1-aminoindane optiquement pur pour préparer de la rasagiline ou un sel pharmaceutiquement acceptable de celle-ci.

15. Procédé pour la préparation de rasagiline ou d'un sel pharmaceutiquement acceptable de celle-ci, comprenant la préparation de (R)-1-aminoindane optiquement pur selon le procédé de l'une quelconque des revendications 1 à 7 et une étape supplémentaire dans laquelle ledit (R)-1-aminoindane optiquement pur est converti en rasagiline ou en un sel pharmaceutiquement acceptable de celle-ci.
